# FASCICULE DE BREVET EUROPEEN

(11) **EP 1 595 530 B1**
(45) Date de publication et mention de la délivrance du brevet: **19.03.2008**
(21) Numéro de dépôt: 05290963.7
(22) Date de dépôt: 03.05.2005
(51) Int. Cl.: A61K 8/36, A61K 8/368, A61K 8/34, A61K 8/60, A61K 8/20, A61K 8/46, A61K 8/44, A61Q 5/00, A61Q 5/02

(54) **Composition cosmétique ou dermatologique à base d'un tensioactif, d'un acide monocarboxlique et d'un polyol**
Kosmetische oder dermatologische Zusammensetzung enthaltend ein Tensid, eine Monocarbonsäure und ein Polyol
Cosmetic or dermatological composition containing a surfactant, a monocarboxylic acid and a polyol

(30) Priorité: 10.05.2004 FR 0405031
(43) Date de publication de la demande: 16.11.2005
(73) Titulaire: L'ORÉAL, 75008 Paris (FR)
(72) Inventeur: Müller, Rainer, 76334 Leopoldshafen (DE); Hippe, Thomas, 78112 Fourqueux (FR)
(74) Mandataire: Casalonga, Axel

(56) Documents cités:
- EP-A- 0 587 797
- EP-A- 0 850 634
- EP-A- 1 325 731
- EP-A- 1 329 215
- WO-A-94/09755
- WO-A-95/03781
- WO-A-96/11572
- DE-A- 10 034 636
- US-A1- 2003 022 799
- US-B2- 6 447 793
- DATABASE WPI Section Ch, Week 199524 Derwent Publications Ltd., London, GB; Class D21, AN 1995-183738 XP002307262 & RU 2 020 929 C1 (CHEM IND RES INST) 15 octobre 1994 (1994-10-15)
- DATABASE WPI Section Ch, Week 199215 Derwent Publications Ltd., London, GB; Class B02, AN 1992-120842 XP002342484 & JP 04 066508 A (SUNTORY LTD) 2 mars 1992 (1992-03-02)
- PATENT ABSTRACTS OF JAPAN vol. 2000, no. 05, 14 septembre 2000 (2000-09-14) & JP 2000 063242 A (SHISEIDO CO LTD), 29 février 2000 (2000-02-29)

## Description

La présente invention est relative à de nouvelles compositions cosmétiques ou dermatologiques, notamment capillaires, comprenant au moins un tensioactif, au moins un acide monocarboxylique, au moins un polyol et au moins un sel, différent du sel d'acide monocarboxylique et du tensioactif. Elle vise également l'utilisation de ces compositions en cosmétique, et l'utilisation de ces compositions pour la préparation d'une composition dermatologique pour le traitement des désordres liés à Aspergillus niger.

Les compositions cosmétiques qui contiennent une teneur en eau importante, et notamment les shampooings, doivent contenir des agents conservateurs pour d'éviter la croissance de microorganismes. En particulier, les moisissures et l'une d'entre elles, l'Aspergillus niger, sont particulièrement difficiles à éliminer. Ainsi, des concentrations importantes en agents conservateurs sont nécessaires.

Toutefois, les agents conservateurs possèdent parfois un potentiel allergique. Il est par conséquent souhaitable de diminuer la concentration en agents conservateurs.

Il apparaît ainsi nécessaire de disposer de compositions qui permettent d'éviter la croissance de microorganismes, tout en présentant une concentration en agents conservateurs moindre.

On connaît du document EP 1 325 731 un agent microbicide comprenant l'association de l'acide anisique avec un polyol et un tensioactif cationique.

Toutefois, ces compositions à base d'acide anisique sont peu stables.

Le document RU 2 020 99 décrit des compositions à base de tensioactif, de glycérol, de NaCl et d'acide salicylique.

Toutefois, ces compositions, ayant une activité de l'eau à 25°C supérieure à 0,96, présentent une activité antimicrobienne insuffisante.

La Demanderesse vient de découvrir de façon surprenante que des compositions comprenant au moins un tensioactif, au moins un acide monocarboxylique particulier, au moins un polyol, au moins un sel, différent du sel d'acide monocarboxylique et du tensioactif présent dans la composition à une teneur comprise entre 1 et 10% en poids du poids total de la composition, bornes comprises avec une activité de l'eau à 25°C inférieure à 0,96, et/ou avec une teneur en polyol comprise entre 3 et 20% en poids, présentaient une activité satisfaisante contre les microorganismes.

Elle a en outre constaté que les shampooings à base de cette composition possédaient des propriétés inchangées, et notamment les propriétés physico-chimiques et cosmétiques, en particulier la viscosité et la qualité de la mousse.

Enfin, elle a découvert que ces compositions présentaient une activité renforcée contre Aspergillus niger, et permettaient ainsi de préparer des shampooings d'aspect esthétique et sain, et qu'elles permettaient de combattre les désordres liés à Aspergillus niger, en particulier les irritations du cuir chevelu et les maladies liées à ce microorganisme.

La présente invention a donc pour objet une composition cosmétique ou dermatologique, notamment capillaire, caractérisée en ce qu'elle comprend, dans un milieu physiologiquement acceptable :
a) au moins un tensioactif ;
b) au moins un acide monocarboxylique choisi parmi l'acide benzoïque, l'acide salicylique, l'acide sorbique, l'acide déhydroacétique, l'acide propionique et l'acide caproique, ou un de ses sels, et présent dans la composition à une teneur comprise entre 0,1 et 1% en poids du poids total de la composition, bornes comprises,
c) au moins un polyol, de préférence non éthérifié, et de masse moléculaire inférieure à 500, présent dans la composition à une teneur comprise entre 3 et 20% en poids du poids total de la composition, bornes comprises, et
au moins un sel, différent du sel d'acide monocarboxylique et du tensioactif présent dans la composition à une teneur comprise entre 3 et 10% en poids du poids total de la composition, bornes comprises.
L'activité de l'eau à 25 °C d'une telle composition est inférieure ou égale à 0,96.

Un autre objet de l'invention concerne un procédé cosmétique capillaire mettant en oeuvre les compositions selon l'invention.

L'invention a également pour objet l'utilisation des compositions selon l'invention pour le traitement cosmétique des cheveux et du cuir chevelu.

Elle a également pour objet l'utilisation des compositions selon l'invention pour la préparation d'une composition dermatologique destinée au traitement des désordres liés à Aspergillus niger.

D'autres caractéristiques, aspects, objets et avantages de l'invention apparaîtront encore plus clairement à la lecture de la description et de l'exemple qui suivent.

Les compositions selon l'invention comprennent au moins un acide monocarboxylique particulier, ou un de ses sels, minéraux ou organiques.

De préférence, l'acide monocarboxylique est choisi parmi l'acide benzoïque, l'acide salicylique, l'acide sorbique et l'acide déhydroacétique, et encore de préférence parmi l'acide benzoïque, l'acide sorbique et l'acide déhydroacétique.

Encore plus préférentiellement, l'acide monocarboxylique est non hydroxylé.

L'acide monocarboxylique est de préférence présent dans la composition à une teneur comprise entre 0,15 et 0,5% en poids du poids total de la composition, bornes comprises.

On peut utiliser tout polyol de masse moléculaire inférieure à 500 dans les compositions selon l'invention. Par polyol au sens de l'invention, on entend tout composé possédant au moins deux fonctions OH libres. Le polyol est de préférence non éthérifié.

Le ou les polyols peuvent notamment être choisis parmi le sorbitol, le glucose, le fructose, le xylose, le tréhalose, le sucrose, le maltose, le lactose, les polyéthylène glycols, les diols ou triols en C₃-C₈, notamment le butane diol.

On préfère les polyols non éthérifiés et les polyols non esterifiés.

Les polyols particulièrement préférés sont le glycérol et le 1,2-propylène glycol, et de préférence le glycérol.

Le ou les polyols sont de préférence présents dans la composition à une teneur comprise entre 1 et 10%, et encore de préférence entre 4 et 20%, en poids du poids total de la composition, bornes comprises.

Le ou les tensioactifs peuvent être indifféremment choisis, seuls ou en mélanges, au sein des tensioactifs anioniques, amphotères, non ioniques, zwittérioniques et cationiques.

Les tensioactifs convenant à la mise en oeuvre de la présente invention sont notamment les suivants :

### (i) Tensioactif(s) anionique(s) :

A titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkyl(C₆-C₂₄) sulfosuccinates, les alkyl(C₆-C₂₄) éthersulfosuccinates, les alkyl(C₆-C₂₄) amidesulfosuccinates ; les alkyl(C₆-C₂₄) sulfoacétates ; les acyl(C₆-C₂₄) sarcosinates et les acyl(C₆-C₂₄) glutamates. On peut également utiliser les esters d'alkyl(C₆-C₂₄)polyglycosides carboxyliques tels que les alkylglucoside citrates, les alkylpolyglycoside tartrate et les alkylpolyglycoside sulfosuccinates., les alkylsulfosuccinamates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyllactylates dont le radical acyle comporte 8 à 20 atomes de carbone. On peut également utiliser les acides d'alkyl D galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols polyéthoxylés, polypropoxylés, ayant une chaîne grasse comportant par exemple 8 à 18 atomes de carbone, le nombre de groupements oxyde d'éthylène ou oxyde de propylène pouvant aller notamment de 2 à 50. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂ -CONHCH₂CH₂ -N(R₃)(R₄)(CH₂COO⁻)

dans laquelle : R₂ désigne un radical alkyle linéaire ou ramifié en C₅-C₂₀ provenant par exemple d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₂'-CONHCH₂CH₂-N(B)(C)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO₃H
R₂' désigne un radical alkyle, linéaire ou ramifié, saturé ou non, en C₅-C₂₀, d'un acide R₉ -COOH présent par exemple dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL® C2M concentré par la société RHODIA CHIMIE.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées ; les sels d'ammonium quaternaire tels que les chlorures ou les bromures de tétraalkylammonium, d'alkylamidoalkyltrialkylammonium, de trialkylbenzylammonium, de trialkylhydroxyalkyl-ammonium ou d'alkylpyridinium; les dérivés d'imidazoline ; ou les oxydes d'amines à caractère cationique.

Le ou les tensioactifs peuvent être présents dans la composition à une teneur comprise entre 0,01 et 40%, et de préférence entre 0,5 et 30%, et encore plus préférentiellement entre 4 et 25% en poids du poids total de la composition, bornes comprises.

La composition selon l'invention comprend en outre au moins un sel, différent du sel d'acide monocarboxylique et du tensioactif mentionnés ci-dessus. Ainsi, l'activité antifongique de la composition est renforcée.

Le ou les sels peuvent être organiques ou minéraux.

Parmi les sels, on peut citer les sels d'acides minéraux ou organiques en C1-C7 autres que les sels d'acides monocarboxyliques et les tensioactifs décrits ci-dessus. On peut également citer les sels organiques d'ammonium ou d'une amine primaire, éventuellement hydroxylée.

On peut utiliser les sels métalliques. Les métaux peuvent notamment être choisis parmi les métaux alcalins, les métaux alcalinoterreux, et les métaux de transition.

Les métaux sont de préférence mono- ou divalents. Les métaux préférés sont choisis parmi Na, Ca, Li, K, Mg, Cu et Zn.

Les sels préférés sont choisis parmi le chlorure de sodium, le chlorure de potassium, le chlorure de lithium, le chlorure d'ammonium, le sulfate de sodium, le sulfate de magnésium, l'acétate de sodium, le sulfate de cuivre et le citrate de sodium.

Le ou les sels sont présents dans la composition à une teneur comprise entre 1 et 10%, de préférence entre 1 et 5%, et encore de préférence entre 1 et 4% en poids du poids total de la composition, bornes comprises.

Le rapport en masse sel/polyol est de préférence compris entre 1:1 et 1:10.

L'activité de l'eau à 25°C de la composition est de préférence inférieure ou égale à 0,95, et de préférence comprise entre 0,75 et 0,95. Cette activité peut être mesurée à l'aide de l'instrument Hygroscop BT-RS1 de la société Rotronic. L'activité de l'eau est le rapport entre la pression de vapeur d'un produit et la pression de vapeur de l'eau pure, à la même température.

Le pH de la composition est de préférence inférieur à 7, et encore de préférence inférieur à 6.

Le milieu physiologiquement acceptable est généralement constitué par de l'eau ou par un mélange d'eau et d'au moins un solvant organique acceptable d'un point de vue physiologique. Parmi ces solvants, on peut mentionner les alcools en C1-C4.

Parmi ces alcools, on peut citer l'éthanol, l'isopropanol. L'éthanol est particulièrement préféré.

La concentration en solvant(s) organique est de préférence inférieure à 30%, et encore de préférence à 10% en poids du poids total de la composition.

La composition selon l'invention peut contenir en outre au moins un additif choisi parmi les parfums, les filtres, les conservateurs, les protéines, les vitamines, les polymères non ioniques, anioniques, cationiques, amphotères ou zwitterioniques, les huiles minérales, végétales ou synthétiques, et tout autre additif classiquement utilisé dans les compositions cosmétiques, tels que les agents antipelliculaires, les agents anti-chute, les colorants, les pigments, les réducteurs.

Ces additifs sont présents dans la composition à des teneurs avantageusement comprises entre 0,001 et 20% en poids du poids total de la composition, bornes comprises. La quantité précise de chaque additif est fonction de sa nature et est déterminée facilement par l'homme du métier, et dépendra de l'application capillaire choisie.

Bien entendu, l'homme du métier veillera à choisir le ou les additifs de manière que les propriétés avantageuses de la composition conforme à l'invention ne soient pas, ou substantiellement pas, altérées par l'addition envisagée.

Les compositions selon l'invention peuvent se présenter sous forme de lotions, de shampooings, de mousses, de crèmes, de gels, de sticks, de sprays, de baumes, de savons, de préférence sous forme de shampooings.

Les compositions de l'invention peuvent être utilisées pour la fabrication de nombreux produits capillaires, comme par exemple, des produits pour la fixation et/ou le maintien des cheveux, des produits de conditionnement, comme des formulations de brillance ou encore des produits de soin des cheveux.

Ces compositions peuvent être conditionnées sous diverses formes, notamment dans des flacons pompes ou dans des récipients aérosols, afin d'assurer une application de composition sous forme vaporisée ou sous forme de mousse.

Lorsque la composition selon l'invention est conditionnée sous forme d'aérosol, elle comprend au moins un agent propulseur qui peut être choisi parmi les hydrocarbures volatils tels que le n-butane, le propane, l'isobutane, le pentane, un hydrocarbure halogéné et leurs mélanges. On peut également utiliser en tant qu'agent propulseur le gaz carbonique, le protoxyde d'azote, le diméthyléther (DME), l'azote, l'air comprimé. On peut aussi utiliser des mélanges de propulseurs. De préférence, on utilise le diméthyléther.

Avantageusement, l'agent propulseur est présent à une concentration comprise entre 5 et 90% en poids par rapport au poids total au poids total de la composition dans le dispositif aérosol, et plus particulièrement à une concentration comprise entre 10 et 60%, bornes comprises.

L'invention a également pour objet l'utilisation d'une composition telle que définie ci-dessus pour le traitement cosmétique des cheveux et du cuir chevelu.

L'invention a encore pour objet l'utilisation d'une composition telle que définie ci-dessus, comme composition cosmétique antifongique.

L'invention concerne aussi l'utilisation d'une composition telle que définie ci-dessus pour la préparation d'une composition dermatologique destinée au traitement des désordres liés à Aspergillus niger.

La présente invention a également pour objet un procédé de traitement cosmétique dans lequel la composition cosmétique selon l'invention est appliquée sur les cheveux, humides ou secs, cette application étant éventuellement suivie d'un rinçage.

Dans une forme de réalisation préférée de l'invention, les compositions selon l'invention sont utilisées comme shampooings pour le lavage et le traitement des cheveux et du cuir chevelu.

Ils sont appliqués, dans ce cas-là, sur des cheveux humides ou secs, dans des quantités efficaces pour les laver, cette application étant suivie d'un rinçage.

Les exemples suivants sont destinés à illustrer l'invention.

### Exemple 1

On a préparé des shampooings de compositions 1 et 2 suivantes :

| Composition | **1** | **2** |
|---|---|---|
| Lauryl Ether Sulfate de Sodium à 70% de matière active | 15 g | 15 g |
| Cocamidopropyl Betaine à 37% de matière active | 6,4 g | 6,4 g |
| Polyquaternium-10 | 0,5 g | 0,5 g |
| Chlorure de Sodium | 3,8 g | - |
| Glycérine | 4 g | - |
| Benzoate de Sodium | 0,3 g | 0,3 g |
| Acide Citrique | 0,04 g | 0,06 g |
| Eau qsp | 100 g | 100 g |
| pH | 5,7 | 5,6 |
| Activité de l'eau | 0,95 | 0,99 |
| Escherichia coli | < 200 | 3,1E+05 |
| Pseudomonas aeruginosa | < 200 | 2,5E+04 |
| Staphylococcus aureus | < 200 | < 200 |
| Enterococcus faecalis | < 200 | < 200 |
| Bacillus cereus | 2,8E+06 | 2,8E+06 |
| Candida albicans | < 200 | < 200 |
| Aspergillus niger | < 200 | 1,1E+05 |
| (Nombre de germes après 7 jours) | | |

On a déterminé le nombre de germes de différents microorganismes, et notamment d'Aspergillus niger, après 7 jours. On a constaté que la composition 1 selon l'invention procurait une bonne protection antimicrobienne, et en particulier une bonne protection antifongique.

### Exemple 2

On a préparé des shampooings de compositions 3, 4 et 5 suivantes :

| Composition | **3** | **4** |
|---|---|---|
| Lauryl Ether Sulfate de Sodium à 70% de matière active | 20 g | 15 g |
| Cocamidopropyl Betaine à 37% de matière active | 6,4 g | 6,4 g |
| Chlorure de Sodium | 3,5 g | 3,8 g |
| Glycérine | 3 g | 4 g |
| Héxylène glycol | 1g | - |
| Fructose | 2 g | - |
| Acide salicylique | - | 0,3 g |
| Déhydroacétate de sodium | 0,2 g | - |
| Eau qsp | 100 g | 100 g |
| pH | 6,1 | 5,1 |
| Activité de l'eau | 0,95 | 0,95 |

## Revendications

1. Composition cosmétique ou dermatologique, notamment capillaire, **caractérisée en ce qu**'elle comprend, dans un milieu physiologiquement acceptable,
a) au moins un tensioactif ;
b) au moins un acide monocarboxylique choisi parmi l'acide benzoïque, l'acide salicylique, l'acide sorbique, l'acide déhydroacétique, l'acide propionique et l'acide caproique, ou un de ses sels, et présent dans la composition à une teneur comprise entre 0,1 et 1% en poids du poids total de la composition, bornes comprises,
c) au moins un polyol de masse moléculaire inférieure à 500, présent dans la composition à une teneur comprise entre 3 et 20% en poids du poids total de la composition, bornes comprises ; et
d) au moins un sel, différent du sel d'acide monocarboxylique et du tensioactif, présent dans la composition à une teneur comprise entre 1 et 10% en poids du poids total de la composition, bornes comprises.

2. Composition selon la revendication 1, **caractérisée en ce que** l'acide monocarboxylique est choisi parmi l'acide benzoïque, l'acide salicylique, l'acide sorbique et l'acide déhydroacétique.

3. Composition selon la revendication 2, **caractérisée en ce que** l'acide monocarboxylique est choisi parmi l'acide benzoïque, l'acide sorbique et l'acide déhydroacétique.

4. Composition selon l'une quelconque des revendications précédentes, **caractérisée en ce que** l'acide monocarboxylique est présent dans la composition à une teneur comprise entre 0,15 et 0,5% en poids du poids total de la composition, bornes comprises.

5. Composition selon la revendication 1, **caractérisée en ce que** le polyol est choisi parmi le sorbitol, le glucose, le fructose, le xylose, le tréhalose, le sucrose, le maltose, le lactose, les polyéthylène glycols, les diols ou triols en C₃-C₈.

6. Composition selon la revendication 5, **caractérisée en ce que** le polyol est choisi parmi le glycérol et le 1,2-propylène glycol.

7. Composition selon la revendication 6, **caractérisée en ce que** le polyol est le glycérol.

8. Composition selon l'une quelconque des revendications précédentes, **caractérisée en ce que** le polyol est présent dans la composition à une teneur comprise entre 4 et 20%, de préférence entre 4 et 10% en poids du poids total de la composition, bornes comprises.

9. Composition selon l'une quelconque des revendications précédentes, **caractérisée en ce que** le sel est choisi parmi les sels d'acides minéraux ou organiques en C1-C7 et les sels organiques d'ammonium ou d'une amine primaire, éventuellement hydroxylée.

10. Composition selon l'une quelconque des revendications 1 à 8, **caractérisée en ce que** le sel est un sel d'un métal choisi parmi Na, K, Li, Ca, Mg, Cu et Zn.

11. Composition selon l'une quelconque des revendications précédentes, **caractérisée en ce que** le sel est choisi parmi le chlorure de sodium, le chlorure de potassium, le chlorure de lithium, le chlorure d'ammonium, le sulfate de sodium, le sulfate de magnésium, l'acétate de sodium, le sulfate de cuivre et le citrate de sodium.

12. Composition selon l'une des revendications précédentes, **caractérisée en ce que** le sel est présent dans la composition à des teneurs comprises entre 1 et 5%, et de préférence entre 1 et 4% en poids du poids total de la composition, bornes comprises.

13. Composition selon l'une quelconque des revendications précédentes, **caractérisée en ce que** le rapport en masse sel /polyol est compris entre 1:1 et 1:10.

14. Composition selon l'une quelconque des revendications précédentes, **caractérisée en ce que** l'activité de l'eau de la composition à 25 °C est comprise entre 0,75 et 0,95.

15. Composition selon l'une quelconque des revendications précédentes, **caractérisée en ce que** le pH de la composition est inférieur à 7, et de préférence inférieur à 6.

16. Composition selon l'une quelconque des revendications précédentes, **caractérisée en ce que** le milieu physiologiquement acceptable est constitué par de l'eau ou par un mélange d'eau et d'au moins un solvant organique choisi parmi les alcools en C1-C4.

17. Composition selon l'une quelconque des revendications précédentes, **caractérisée en ce qu**'elle comprend en outre au moins un additif choisi parmi les parfums, les filtres, les conservateurs, les protéines, les vitamines, les polymères non ioniques, anioniques, cationiques, amphotères ou zwitterioniques, les huiles minérales, végétales ou synthétiques, les agents antipelliculaires, les agents anti-chute, les colorants, les pigments, les réducteurs.

18. Composition selon l'une quelconque des revendications précédentes, **caractérisée en ce qu'**elle se présente sous forme d'un shampooing.

19. Utilisation d'une composition selon l'une des revendications 1 à 18 pour le traitement cosmétique des cheveux et du cuir chevelu.

20. Utilisation d'une composition selon l'une des revendications 1 à 18 comme composition cosmétique antifongique.

21. Utilisation d'une composition selon l'une des revendications 1 à 18 pour la préparation d'une composition dermatologique destinée au traitement des désordres liés à Aspergillus niger.

22. Procédé de traitement cosmétique, **caractérisé en ce qu**'on applique sur les cheveux une composition cosmétique selon l'une des revendications 1 à 18.

## Claims

1. Cosmetic or dermatological composition, especially a haircare composition, **characterized in that** it comprises, in a physiologically acceptable medium:
a) at least one surfactant;
b) at least one monocarboxylic acid chosen from benzoic acid, salicylic acid, sorbic acid, dehydroacetic acid, propionic acid and caproic acid, or a salt thereof, and present in the composition in a content of between 0.1% and 1% by weight relative to the total weight of the composition, limits inclusive,
c) at least one polyol with a molecular weight of less than 500, present in the composition in a content of between 3% and 20% by weight relative to the total weight of the composition, limits inclusive; and
d) at least one salt other than the salt of monocarboxylic acid and than the surfactant, which is present in the composition in a content of between 1% and 10% by weight relative to the total weight of the composition, limits inclusive.

2. Composition according to Claim 1, **characterized in that** the monocarboxylic acid is chosen from benzoic acid, salicylic acid, sorbic acid and dehydroacetic acid.

3. Composition according to Claim 2, **characterized in that** the monocarboxylic acid is chosen from benzoic acid, sorbic acid and dehydroacetic acid.

4. Composition according to any one of the preceding claims, **characterized in that** the monocarboxylic acid is present in the composition in a content of between 0.15% and 0.5% by weight relative to the total weight of the composition, limits inclusive.

5. Composition according to Claim 1, **characterized in that** the polyol is chosen from sorbitol, glucose, fructose, xylose, trehalose, sucrose, maltose, lactose, polyethylene glycols and C₃-C₈ diols or triols.

6. Composition according to Claim 5, **characterized in that** the polyol is chosen from glycerol and 1,2-propylene glycol.

7. Composition according to Claim 6, **characterized in that** the polyol is glycerol.

8. Composition according to any one of the preceding claims, **characterized in that** the polyol is present in the composition in a content of between 4% and 20% and preferably between 4% and 10% by weight relative to the total weight of the composition, limits inclusive.

9. Composition according to any one of the preceding claims, **characterized in that** the salt is chosen from mineral and C1-C7 organic acid salts and organic salts of ammonium or of a primary amine, which is optionally hydroxylated.

10. Composition according to any one of Claims 1 to 8, **characterized in that** the salt is a salt of a metal chosen from Na, K, Li, Ca, Mg, Cu and Zn.

11. Composition according to one of the preceding claims, **characterized in that** the salt is chosen from sodium chloride, potassium chloride, lithium chloride, ammonium chloride, sodium sulfate, magnesium sulfate, sodium acetate, copper sulfate and sodium citrate.

12. Composition according to one of the preceding claims , **characterized in that** the salt is present in the composition in contents of between 1% and 5% and preferably between 1% and 4% by weight relative to the total weight of the composition, limits inclusive.

13. Composition according to any one of the preceding claims , **characterized in that** the salt/polyol weight ratio is between 1:1 and 1:10.

14. Composition according to any one of the preceding claims, **characterized in that** the water activity of the composition at 25°C is between 0.75 and 0.95.

15. Composition according to any one of the preceding claims, **characterized in that** the pH of the composition is less than 7 and preferably less than 6.

16. Composition according to any one of the preceding claims, **characterized in that** the physiologically acceptable medium consists of water or of a mixture of water and of at least one organic solvent chosen from C1-C4 alcohols.

17. Composition according to any one of the preceding claims, **characterized in that** it also comprises at least one additive chosen from fragrances, screening agents, preserving agents, proteins, vitamins, nonionic, anionic, cationic, amphoteric or zwitterionic polymers, mineral, plant or synthetic oils, antidandruff agents, hair-loss counteractants, dyes, pigments and reducing agents.

18. Composition according to any one of the preceding claims, **characterized in that** it is in the form of a shampoo.

19. Use of a composition according to one of Claims 1 to 18, for the cosmetic treatment of the hair and the scalp.

20. Use of a composition according to one of Claims 1 to 18, as an antifungal cosmetic composition.

21. Use of a composition according to one of Claims 1 to 18, for the preparation of a dermatological composition for treating disorders related to Aspergillus niger.

22. Cosmetic treatment process, **characterized in that** a cosmetic composition according to one of Claims 1 to 18 is applied to the hair.

## Patentansprüche

1. Kosmetische oder dermatologische Zusammensetzung und insbesondere Zusammensetzung für die Haarbehandlung, **dadurch gekennzeichnet, dass** sie in einem physiologisch akzeptablen Medium enthält:
a) mindestens einen grenzflächenaktiven Stoff,
b) mindestens eine Monocarbonsäure, die unter Benzoesäure, Salicylsäure, Sorbinsäure, Dehydroessigsäure, Propionsäure und Capronsäure oder einem ihrer Salze ausgewählt ist und in der Zusammensetzung in einer Menge von 0,1 bis 1 Gew.-% des Gesamtgewichts der Zusammensetzung enthalten ist, wobei die Grenzen eingeschlossen sind,
c) mindestens ein Polyol mit einer Molmasse unter 500, das in der Zusammensetzung in einer Menge von 3 bis 20 Gew.-% des Gesamtgewichts der Zusammensetzung enthalten ist, wobei die Grenzen eingeschlossen sind;
d) mindestens ein Salz, das von dem Salz der Monocarbonsäure und dem grenzflächenaktiven Stoff verschieden ist und in der Zusammensetzung in einer Menge von 1 bis 10 Gew.-% des Gesamtgewichts der Zusammensetzung enthalten ist, wobei die Grenzen eingeschlossen sind.

2. Zusammensetzung nach Anspruch 1, **dadurch gekennzeichnet, dass** die Monocarbonsäure unter Benzoesäure, Salicylsäure, Sorbinsäure und Dehydroessigsäure ausgewählt ist.

3. Zusammensetzung nach Anspruch 2, **dadurch gekennzeichnet, dass** die Monocarbonsäure unter Benzoesäure, Sorbinsäure und Dehydroessigsäure ausgewählt ist.

4. Zusammensetzung nach einem der vorhergehenden Ansprüche, **dadurch gekennzeichnet, dass** die Monocarbonsäure in der Zusammensetzung in einer Menge von 0,15 bis 0,5 Gew.-% des Gesamtgewichts der Zusammensetzung enthalten ist, wobei die Grenzen eingeschlossen sind.

5. Zusammensetzung nach Anspruch 1, **dadurch gekennzeichnet, dass** das Polyol unter Sorbitol, Glucose, Fructose, Xylose, Trehalose, Saccharose, Maltose, Lactose, Polyethylenglycolen, Diolen oder Triolen mit 3 bis 8 Kohlenstoffatomen ausgewählt ist.

6. Zusammensetzung nach Anspruch 5, **dadurch gekennzeichnet, dass** das Polyol unter Glycerol und 1,2-Propylenglycol ausgewählt ist.

7. Zusammensetzung nach Anspruch 6, **dadurch gekennzeichnet, dass** das Polyol das Glycerol ist.

8. Zusammensetzung nach einem der vorhergehenden Ansprüche, **dadurch gekennzeichnet, dass** das Polyol in der Zusammensetzung in einer Menge von 4 bis 20 Gew.-% und vorzugsweise 4 bis 10 Gew.-% des Gesamtgewichts der Zusammensetzung enthalten ist, wobei die Grenzen eingeschlossen sind.

9. Zusammensetzung nach einem der vorhergehenden Ansprüche, **dadurch gekennzeichnet, dass** das Salz unter den Salzen von anorganischen Säuren und organischen Säuren mit 1 bis 7 Kohlenstoffatomen und den organischen Ammoniumsalzen oder organischen Salzen eines primären, gegebenenfalls hydroxylierten Amins ausgewählt ist.

10. Zusammensetzung nach einem der Ansprüche 1 bis 8, **dadurch gekennzeichnet, dass** das Salz ein Salz eines Metalls ist, das unter Na, K, Li, Ca, Mg, Cu und Zn ausgewählt ist.

11. Zusammensetzung nach einem der vorhergehenden Ansprüche, **dadurch gekennzeichnet, dass** das Salz unter Natriumchlorid, Kaliumchlorid, Lithiumchlorid, Ammoniumchlorid, Natriumsulfat, Magnesiumsulfat, Natriumacetat, Kupfersulfat und Natriumcitrat ausgewählt ist.

12. Zusammensetzung nach einem der vorhergehenden Ansprüche, **dadurch gekennzeichnet, dass** das Salz in der Zusammensetzung in Mengenanteilen von 1 bis 5 % und vorzugsweise 1 bis 4 Gew.-% des Gesamtgewichts der Zusammensetzung enthalten ist, wobei die Grenzen eingeschlossen sind.

13. Zusammensetzung nach einem der vorhergehenden Ansprüche, **dadurch gekennzeichnet, dass** das Masseverhältnis Salz/Polyol im Bereich von 1:1 1 bis 1:10 liegt.

14. Zusammensetzung nach einem der vorhergehenden Ansprüche, **dadurch gekennzeichnet, dass** die Wasseraktivität der Zusammensetzung bei 25 °C im Bereich von 0,75 bis 0,95 liegt.

15. Zusammensetzung nach einem der vorhergehenden Ansprüche, **dadurch gekennzeichnet, dass** der pH-Wert der Zusammensetzung unter 7 und vorzugsweise unter 6 liegt.

16. Zusammensetzung nach einem der vorhergehenden Ansprüche, **dadurch gekennzeichnet, dass** das physiologisch akzeptable Medium aus Wasser oder einem Gemisch von Wasser und mindestens einem organischen Lösungsmittel besteht, das unter den C₁₋₄-Alkoholen ausgewählt ist.

17. Zusammensetzung nach einem der vorhergehenden Ansprüche, **dadurch gekennzeichnet, dass** sie ferner mindestens einen Zusatzstoff enthält, der unter den Parfums, Filtern, Konservierungsmitteln, Proteinen, Vitaminen, nichtionischen, anionischen, kationischen, amphoteren oder zwitterionischen Polymeren, Mineralölen, pflanzlichen Ölen oder synthetischen Ölen, Antischuppenmitteln, Wirkstoffen gegen Haarausfall, Farbstoffen, Pigmenten und Reduktionsmitteln ausgewählt ist.

18. Zusammensetzung nach einem der vorhergehenden Ansprüche, **dadurch gekennzeichnet, dass** sie in Form eines Haarwaschmittels vorliegt.

19. Verwendung einer Zusammensetzung nach einem der Ansprüche 1 bis 18 für die kosmetische Behandlung der Haare und der Kopfhaut.

20. Verwendung einer Zusammensetzung nach einem der Ansprüche 1 bis 18 als antimykotische kosmetische Zusammensetzung.

21. Verwendung einer Zusammensetzung nach einem der Ansprüche 1 bis 18 für die Herstellung einer dermatologischen Zusammensetzung, die für die Behandlung von Erkrankungen vorgesehen ist, die mit Aspergillus niger zusammenhängen.

22. Verfahren zur kosmetischen Behandlung, **dadurch gekennzeichnet, dass** auf die Haare eine kosmetische Zusammensetzung nach einem der Ansprüche 1 bis 18 aufgetragen wird.
